# FASCICULE DE BREVET EUROPEEN

(11) **EP 3 537 960 B1**
(45) Date de publication et mention de la délivrance du brevet: **06.01.2021**
(21) Numéro de dépôt: 17804238.8
(22) Date de dépôt: 06.11.2017
(51) Int. Cl.: A61B 5/024, A61B 5/0408

(54) **ÉLECTRODE DE CARDIO-FRÉQUENCEMÈTRE ET PROCÉDÉ DE MESURE DE LA FRÉQUENCE CARDIAQUE**
HERZFREQUENZMONITORELEKTRODE UND VERFAHREN ZUR MESSUNG DER HERZFREQUENZ
HEART RATE MONITOR ELECTRODE AND METHOD FOR MEASURING HEART RATE

(30) Priorité: 09.11.2016 FR 1660836
(43) Date de publication de la demande: 18.09.2019
(73) Titulaire: Arioneo, 41250 Mont Près Chambord (FR)
(72) Inventeur: BUISSON, Thomas, 92140 Clamart (FR); DUBOIS, Guillaume, 75011 Paris (FR); MELLERIO, Erwan, 41250 Mont Près Chambord (FR); YAZIDI, Soumaya, 92800 Puteaux (FR)
(74) Mandataire: INNOV-GROUP
(86) Numéro de dépôt international: PCT/FR2017/053028
(87) Numéro de publication internationale: WO 2018/087461

(56) Documents cités:
- EP-A1- 0 571 120
- US-A- 5 305 746
- US-A1- 2013 023 748
- US-B1- 6 510 333

## Description

L'invention concerne le domaine de la mesure de la fréquence cardiaque. Notamment, l'invention concerne une électrode et un procédé de mesure de la fréquence cardiaque d'animaux comportant des poils.

On connaît, par exemple par le document FR 2 531 330, un dispositif pour mesurer la fréquence cardiaque comportant une cuvette avec une paroi latérale souple, dans laquelle est placée une éponge imbibée d'un gel ou de toute autre matière malléable bonne conductrice de l'électricité. Cette éponge permet ainsi la conduction d'un signal électrique entre l'animal et une pastille connectée à un câble conducteur de l'électricité.

La mesure et le traitement d'un signal représentant la fréquence cardiaque ne donnent pas toujours un résultat fiable. La qualité du résultat issu de ce type de mesure et de traitement dépend beaucoup de la qualité de la transmission du signal électrique se propageant en surface de la peau de l'animal, entre celle-ci et l'électrode destinée à recueillir ce signal. Un but de l'invention est d'améliorer les électrodes de l'art antérieur afin d'obtenir un meilleur rapport signal sur bruit. Le document US2013/023748 décrit une électrode selon le préambule de la revendication 1.

A cette fin, il est prévu selon l'invention une électrode de cardio-fréquencemètre comprenant
- des pointes électriquement conductrices s'étendant chacune, d'une extrémité libre à un circuit électriquement conducteur reliant électriquement les pointes les unes aux autres,
- une plaque de matériau absorbant, souple, comportant une surface de contact et pouvant changer de forme élastiquement entre
   ∘ une forme au moins partiellement expansée dans laquelle la plaque de matériau absorbant est apte à retenir un liquide et
   ∘ une forme au moins partiellement compressée, dans laquelle la plaque de matériau absorbant occupe un volume moindre que sous forme expansée et dans laquelle au moins une partie d'un liquide contenu dans la plaque de matériau absorbant lorsqu'il est sous forme au moins partiellement expansée, est libéré.

L'extrémité libre d'au moins certaines des pointes affleure sur la surface de contact de la plaque de matériau absorbant ou dépasse de celle-ci lorsque la plaque de matériau absorbant est sous une forme au moins partiellement compressée et libère au moins une partie du liquide qu'elle contient sous sa forme au moins partiellement expansée.

Dans ce document, un circuit n'est pas nécessairement constitué d'un ensemble de pistes conductrices. Un flasque conducteur, ou comportant une face conductrice, forme également un circuit en ce sens qu'il connecte électriquement les pointes entre elles.

Grâce à ces dispositions, l'extrémité libre d'au moins certaines des pointes peut venir au contact direct de la peau de l'animal, en passant à travers les poils de celui-ci, pour assurer une conduction optimisée du signal recueilli en surface de sa peau. Le fait que le matériau absorbant puisse être déformé élastiquement est aussi avantageux. En effet, l'électrode n'est pas à usage unique et peut être réutilisée de nombreuses fois. Le matériau absorbant peut être réimprégné de liquide avant chaque nouvelle utilisation sans avoir nécessairement à changer le matériau absorbant. Par ailleurs, la compression du matériau absorbant libère le liquide qui peut venir améliorer encore le contact direct entre la peau et l'extrémité libre des pointes passées à travers le pelage de l'animal. Ainsi, la conduction électrique entre la peau et des pointes peut être augmentée, même si l'animal n'est pas en mouvement et n'a pas encore commencé à transpirer. Le rapport signal sur bruit des signaux électriques recueillis par l'électrode se trouve ainsi amélioré et optimisé.

L'électrode selon invention comporte en outre l'une ou l'autre des caractéristiques suivantes considérées isolément ou en combinaison d'une ou plusieurs autres :
- la plaque de matériau absorbant a une épaisseur variant lorsque la plaque de matériau absorbant passe de sa forme au moins partiellement expansée à sa forme au moins partiellement compressée, les pointes s'étendant dans une direction de compression essentiellement parallèle cette épaisseur, et l'électrode comportant un mécanisme élastique permettant de déplacer les pointes relativement à la plaque de matériau absorbant parallèlement à la direction de compression,
- elle comporte un boîtier de forme aplatie avec deux faces principales,
   ∘ une première face principale comportant une ouverture destinée à venir en vis-à-vis d'une peau animale au niveau de laquelle on souhaite recueillir la fréquence cardiaque, cette ouverture permettant le passage de l'extrémité libre des pointes, et
   ∘ une deuxième face principale comportant une surface de pression coopérant avec le mécanisme élastique pour comprimer la plaque de matériau absorbant dans la direction de compression lorsque qu'une pression est exercée sur la deuxième face principale de l'électrode ;
- elle comprend des pointes périphériques, dont les extrémités libres sont disposées essentiellement dans un plan perpendiculaire à la direction de compression et sur un cercle dans ce plan, aucune extrémité libre de pointe n'étant située hors de ce cercle ;
- chaque pointe est entourée d'une portion de la plaque de matériau absorbant ;
- au moins l'extrémité libre des pointes est revêtue d'argent ou d'un alliage d'argent ;
- les pointes, et éventuellement le circuit électriquement conducteur auquel elles sont électriquement reliées, sont réalisés dans un matériau élastique conducteur ; il s'agit par exemple d'un polymère chargé de particules électriquement conductrices.

Selon un autre aspect, l'invention concerne un dispositif cardio-fréquencemètre comprenant un récepteur adapté pour recevoir le signal capté par au moins une électrode comportant l'une ou l'autre des caractéristiques mentionnées ci-dessus, considérées isolément ou en combinaison d'une ou plusieurs autres.

Avantageusement, un émetteur relié électriquement à au moins une électrode et adapté pour communiquer avec le récepteur est alors placé dans un boîtier surélevé par rapport à une bride solidaire d'une électrode, un espace étant ainsi ménagé entre le boîtier et la bride pour le passage d'une sangle destinée à maintenir l'électrode et le boîtier sur un animal, l'électrode étant placée au contact de la peau de celui-ci.

Selon encore un autre aspect l'invention concerne un procédé de mesure de la fréquence cardiaque d'un animal comprenant, les étapes suivantes :
- la fourniture d'une électrode selon l'une des revendications précédentes,
- l'imprégnation au moins partielle de la plaque de matériau absorbant de cette électrode, avec un liquide, et
- le positionnement et le maintien de cette électrode contre la peau de l'animal grâce à des moyens de maintien et de serrage.

Ce procédé comporte éventuellement une étape d'ajustement des moyens de maintien et de serrage sur l'animal, étape au cours de laquelle la plaque de matériau absorbant est comprimée et libère au moins une partie du liquide qu'elle contient.

D'autres caractéristiques et avantages de l'invention apparaîtront à la lecture de la description détaillée qui suit ainsi que sur les dessins annexés. Sur ces dessins :
- La figure 1 représente schématiquement en perspective un exemple de mode de réalisation d'une électrode de cardio-fréquencemètre selon l'invention ;
- La figure 2 représente schématiquement, vu de dessus, l'exemple de mode de réalisation de l'électrode de cardio-fréquencemètre représentée sur la figure 1 ; un encart représente un agrandissement d'une partie de cette figure ;
- La figure 3 représente schématiquement en perspective et de manière éclatée différents éléments de l'électrode des figures 1 et 2 ;
- La figure 4 représente schématiquement coupe, selon la ligne A-A de la figure 2, le mode de réalisation de l'électrode de cardio-fréquencemètre représentée sur les figure 1 à 3 ; et
- Les figures 5 et 6 représentent schématiquement, respectivement de face et de profil, un dispositif cardio fréquencemètre comportant deux électrodes telles que celles représentées sur les figures 1 à 4.

Sur les figures, les mêmes références désignent des éléments identiques ou similaires.

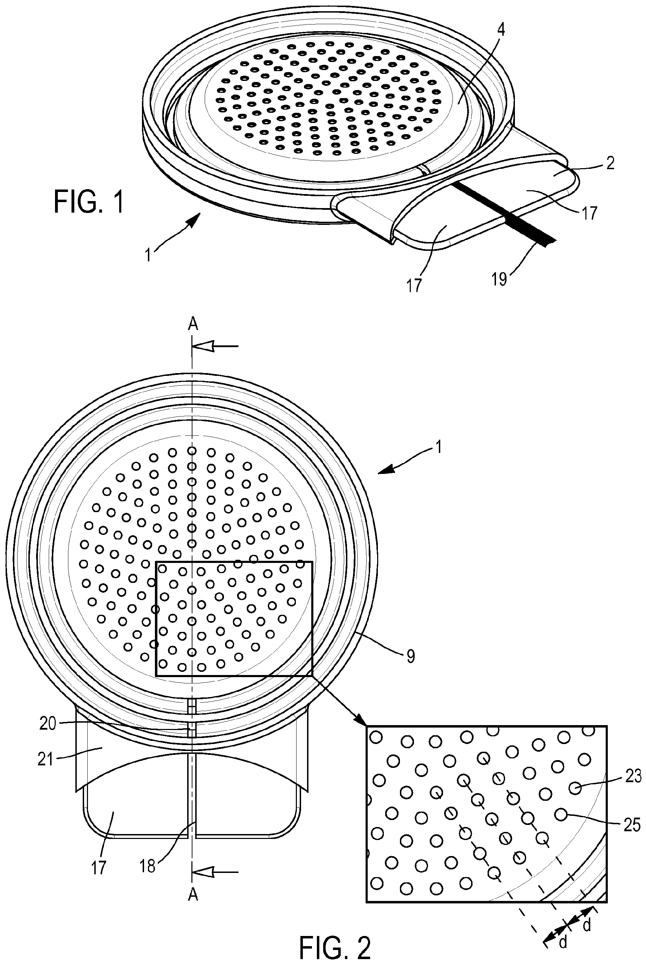

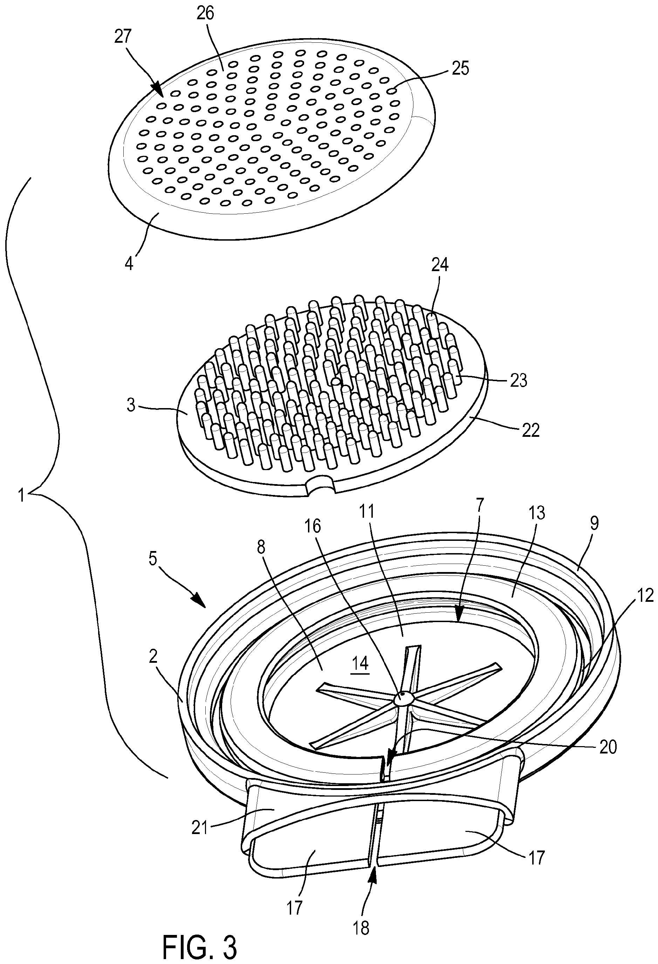

Un exemple de mode de réalisation d'une électrode est décrit ci-dessous en relation avec les figures 1 à 4.

Cette électrode 1 comporte un support 2, une plaque conductrice 3 et une plaque de matériau absorbant 4.

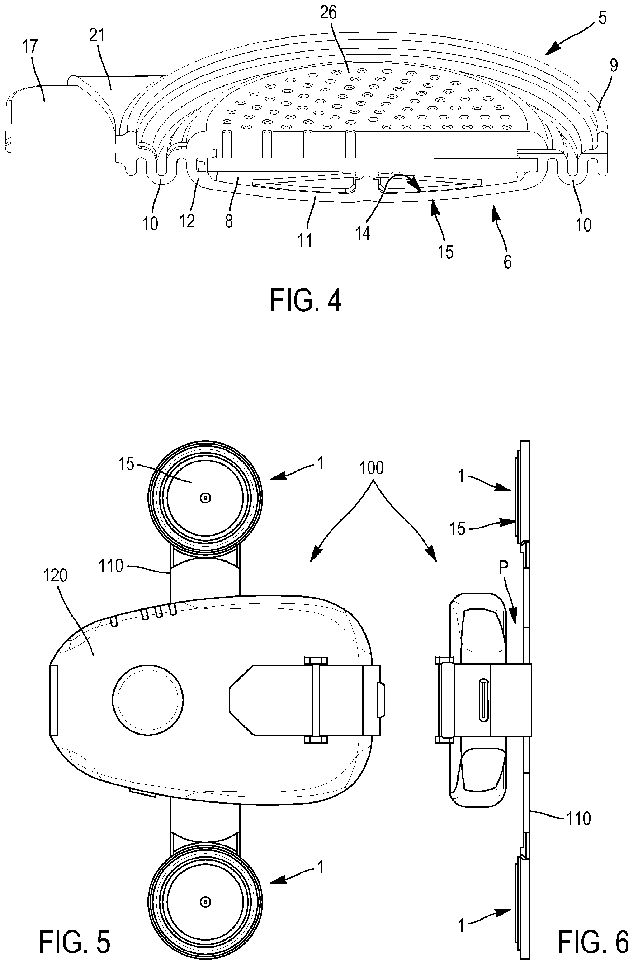

Le support 2 est par exemple réalisé en matière plastique. Il a une forme aplatie avec une première face principale 5 et une deuxième face principale 6 (voir Fig. 4). La première face principale 5 comporte une ouverture 7 destinée à venir en vis-à-vis d'une peau animale au niveau de laquelle on souhaite recueillir la fréquence cardiaque.

Le support 2 comporte une cuvette 8 reliée élastiquement à une monture 9 annulaire externe, par une membrane 10 élastique et souple. La membrane 10 élastique constitue donc un mécanisme, ou moyen, élastique permettant à la cuvette 8 d'être mobile par rapport à la monture 9. La cuvette 8 est essentiellement ronde. Elle comporte un fond 11 et une paroi 12 s'étendant entre le fond 11 et un bord supérieur 13 délimitant l'ouverture 7. Le fond 11 est concave. Il comporte une surface interne 14 et une surface externe 15. La surface externe 15 constitue une surface de pression. La surface interne 14 comporte une protubérance 16. Cette protubérance 16, dans l'exemple illustré ici, se trouve au centre d'une étoile à six branches renforçant et rigidifiant le fond 11 de la cuvette 8.

Le bord de la monture 9 au contact de la peau se trouve surélevé par rapport au bord supérieur 13 de la cuvette 8 d'une distance comprise par exemple entre 1 à 3 mm et est plus particulièrement égale à 2 mm.

Deux languettes 17 s'étendent radialement vers l'extérieur du support 2, à partir de la monture 9. Un canal 18 est ménagé entre les deux languettes 17 pour le passage d'un câble 19 (voir Fig. 1), par exemple blindé, ou d'un autre élément conducteur. Ce canal 18 qui se prolonge par une fente 20 dans la monture 9, ainsi que dans la paroi 12 et le bord supérieur 13 de la cuvette 8, permet également un écartement des portions du bord supérieur 13 de la cuvette 8, situées de part et d'autre de la fente 10, afin de faciliter la mise en place de la plaque conductrice 3, par clipsage, dans la cuvette 8. Pour maintenir l'écartement des languettes 17, un bandeau 21, éventuellement amovible, s'étend autour des languettes 17, ainsi qu'au-dessus et en dessous d'une portion du canal 18.

La plaque conductrice 3 comporte un circuit électriquement conducteur sous forme d'un flasque 22 essentiellement rond et plat. Des pointes 23 électriquement conductrices s'étendent chacune d'une extrémité libre 24 jusqu'au flasque 22. Ce flasque 22 peut être constitué d'une pièce monobloc en métal conducteur, ou d'un circuit imprimé, ou de tout autre moyen permettant de relier électriquement les pointes conductrices les unes des autres en un circuit électriquement continu. Ainsi, alternativement, les pointes 23, et/ou le circuit électrique 22 auquel elles sont électriquement reliées, sont réalisés dans un polymère chargé de particules électriquement conductrices. Ce polymère est par exemple du polydimethylsiloxane mélangé à de la poudre de carbone (45% du volume total) ayant une taille de grain de 40µm ou des nanotubes de carbone de 40nm (3% du volume total). Des fibres de métal micro ou nanométriques peuvent également être ajoutées au polymère, comme des nano fibres d'argent de 30 nm de diamètre, par exemple.

Chaque pointe conductrice 23 est par exemple constituée d'un cylindre muni au niveau de son extrémité libre 24 d'une surface arrondie. Chaque pointe conductrice 23 est par exemple constituée d'un métal conducteur ou de tout autre matériau permettant une conduction électrique entre son extrémité libre 24 et le circuit électrique ou flasque 22 reliant les pointes conductrices 23 les unes aux autres (par exemple, une pointe conductrice 23 peut être formée d'une matière plastique recouverte d'un ou plusieurs matériaux conducteurs, ou d'un métal conducteur tel que du cuivre recouvert au niveau de l'extrémité libre 24 d'un autre métal conducteur tel que de l'argent ou un alliage d'argent, un polymère chargé de particules conductrices comme mentionné précédemment, etc.). Dans un mode de réalisation, les pointes conductrices 23 sont formées de tronçons de fils d'argent soudés sur un flasque 22 de type circuit imprimé (feuillet de cuivre sur isolant). Dans un autre mode de réalisation, le flasque 22 et les pointes conductrices 23 forment une seule pièce réalisée en fonderie, par exemple en argent (ou l'un de ses alliages), ou un aluminium (ou l'un de ses alliages) recouvert d'argent (ou l'un de ses alliages). Dans encore un autre mode de réalisation, le flasque 22 et les pointes conductrices 23 forment une seule pièce moulée formée d'un polymère chargé de particules conductrices comme mentionné précédemment.

Les pointes conductrices 23 ont par exemple une hauteur à partir du flasque 22 comprise entre 2 et 4 mm, et plus particulièrement égale à 3 mm. Toutes les extrémités libres 24 des pointes conductrices 23 se trouvent dans un même plan. En effet, celles-ci sont destinées à venir au contact de la peau d'un animal et doivent le moins possible être ressenties par celui-ci, afin de ne pas, ou peu, le perturber. Pour cette raison également, les pointes conductrices 23 sont agencées à l'intérieur d'un cercle, de manière relativement uniforme. Préférentiellement, les pointes conductrices 23 sont réparties sur des cercles concentriques. Les cercles sont espacés les uns des autres d'une distance comprise entre 2 et 3 mm et est plus particulièrement égale à 2 mm. La distance d entre deux pointes sur un cercle est comprise entre 2 et 3mm et est préférentiellement égale à 2 mm. Autrement dit, la densité des pointes est par exemple comprise entre une pointe pour 10 mm² et une pointe pour 40 mm², et est plus préférentiellement égale à, ou voisine d'une pointe pour 16 mm². Le cercle dans lequel sont situées toutes les pointes périphériques (les plus externes) a par exemple un rayon compris entre 20 et 40 mm et est plus particulièrement égal à 30 mm. Les pointes périphériques, situées sur ce cercle, sont disposés ainsi de manière uniforme. Cette disposition circulaire et uniforme, sans bord saillant, évite ou limite les sensations perturbantes que pourraient provoquer chez l'animal les pointes conductrices 23.

Le circuit électrique du flasque 22 est relié électriquement à un émetteur (non représenté), par le câble 19 ou tout autre moyen de connectique.

La plaque de matériau absorbant 4 est souple et résiliente. Elle est par exemple constituée de mousse de polyuréthane ou de polypropylène, ou de toute autre matière, par exemple spongieuse, adaptée pour s'imprégner d'un liquide et le relâcher, lorsqu'elle est compressée. La plaque de matériau absorbant 4 a une forme aplatie et ronde. Elle a par exemple un diamètre compris entre 30 et 50 mm de diamètre, plus particulièrement égal à 36 mm, et une épaisseur comprise entre 3 et 6 mm, et plus particulièrement égale à la hauteur des pointes conductrices 23.

La plaque de matériau absorbant 4 est traversée, sur son épaisseur, de trous 25 adaptés, par leur diamètre et leur disposition, pour le passage des pointes conductrices 23. Chaque pointe conductrice 23 est entourée d'une portion de la plaque de matériau absorbant 4. Ainsi, pour l'animal, comme pour la personne manipulant l'électrode 1, l'aspect saillant des pointes conductrices 23 est atténué par la plaque de matériau absorbant 4. Préférentiellement, lorsque la plaque de matériau absorbant 4 n'est pas compressée, l'extrémité libre 24 des pointes conductrices 23 ne ressort pas des trous 25 (éventuellement, l'extrémité libre 24 des pointes conductrices 23 affleure juste à la surface 26 de la face externe 27 de la plaque de matériau absorbant 4). Par contre, cette surface 26 se trouve sensiblement au même niveau que le bord de la monture 9 (voir Fig. 4).

Lorsque la plaque de matériau absorbant 4, la plaque conductrice 3 et le support 2 sont montés ensemble, les pointes conductrices 23 s'étendent dans une direction de compression essentiellement parallèle l'épaisseur de la plaque de matériau absorbant 4.

Le bord de la plaque de matériau absorbant est arrondi et peut être éventuellement au moins en partie glissé sous le bord 13 de la cuvette 8, de manière à maintenir la plaque de matériau absorbant 4 sur le support 2. Une fois la plaque de matériau absorbant 4 en place dans la cuvette 8, celle-ci obture l'ouverture 7 de la cuvette 8.

Un dispositif cardio fréquencemètre 100 est représenté sur les figures 5 et 6. Il comporte en particulier une bride 110 sur laquelle sont montées deux électrodes 1 et un boîtier 120. La bride 110 est par exemple constituée d'une matière plastique souple et comporte des éléments électriquement conducteurs permettant de relier les électrodes 1 à un circuit électronique (non représenté) disposé dans le boîtier 120. Les électrodes 1 et le boîtier 120 peuvent être respectivement connectés à ces éléments électriquement conducteurs par des câbles, éventuellement blindés, ou par toute autre connectique adaptée (par exemple, des mini connecteurs coaxiaux pour signaux haute-fréquence, tels que le modèle U-FL commercialisé par Hirose). Avantageusement, les électrodes 1 et le boîtier 120 peuvent être montés de manière amovible sur la bride 110, pour pouvoir changer individuellement l'un de ces éléments s'il est endommagé ou usé, ou pour retirer temporairement le boîtier 120 sans ôter tout le dispositif 100 de l'animal, etc.

Le boîtier 110 est par exemple constitué d'une matière plastique et est suffisamment étanche pour pouvoir être mouillé sans endommager le circuit électrique qu'il contient. Le circuit électronique qu'il reçoit comporte un émetteur adapté pour communiquer par une liaison sans fil avec un récepteur, des données recueillies par différents capteurs et en particulier par les électrodes 1.

Le boîtier 120 est surélevé par rapport à la bride 110 de manière à ménager un espace P entre le boîtier 120 et la bride 110 pour le passage de moyens de maintien et de serrage. Ces moyens de maintien et de serrage comportent par exemple une sangle (non-représentée) permettant de positionner et maintenir le dispositif 100 sur l'animal. Cet espace ou passage P s'étend transversalement par rapport à la bride 110. Ainsi, la sangle passe sous le boîtier 120 et recouvre les électrodes 1 et plaque que celles-ci au contact de la peau de l'animal.

Préalablement à la mise en place du dispositif 100 sur l'animal, le dispositif 100, ou au moins les plaques de matériau absorbant 4, sont mouillés par exemple avec de l'eau de manière à imprégner et imbiber les plaques de matériau absorbant 4 avec celle-ci. La plaque de matériau absorbant 4 est alors essentiellement sous forme expansée et retient de l'eau. Lors de l'opération de serrage de la sangle, celle-ci étant en contact avec la surface externe 15 de la cuvette 8 exerce alors une pression sur le fond 11 de cette dernière. La cuvette 8 dans son ensemble est déplacée vers la peau de l'animal puisque le bord de la monture 9, au contact de la peau se trouve surélevé par rapport au bord 13 de la cuvette 8. Par ailleurs, comme la surface externe 26 de la plaque de matériau absorbant 4 se trouve sensiblement au même niveau que le bord de la monture 9, la plaque de matériau absorbant 4 est compressée entre le flasque 22 comprimé par la protubérance 16 et la peau. Le matériau absorbant relâche au moins une partie de l'eau qu'il avait absorbée. Les pointes conductrices 23 sortent alors des trous 25 de la plaque de matériau absorbant 4, passent à travers les poils de l'animal et viennent, au moins pour certaines, au contact de son épiderme. La conduction électrique peut ainsi être établie entre l'épiderme et le circuit électronique.

## Revendications

1. Electrode de cardio-fréquencemètre comprenant
- des pointes (23),
- une plaque de matériau absorbant (4), souple, comportant une surface de contact (26) et pouvant changer de forme élastiquement entre
o une forme au moins partiellement expansée dans laquelle la plaque de matériau absorbant (4) est apte à retenir un liquide et
o une forme au moins partiellement compressée, dans laquelle la plaque de matériau absorbant (4) occupe un volume moindre que sous forme expansée et dans laquelle au moins une partie d'un liquide contenu dans la plaque de matériau absorbant (4) lorsqu'il est sous forme au moins partiellement expansée, est libéré,
dans laquelle l'extrémité libre (24) d'au moins certaines des pointes (23) affleure sur la surface de contact (26) de la plaque de matériau absorbant (4) ou dépasse de celle-ci lorsque la plaque de matériau absorbant (4) est sous une forme au moins partiellement compressée et libère au moins une partie du liquide qu'elle contient sous sa forme au moins partiellement expansée,
**caractérisée par le fait que** les pointes (23) sont électriquement conductrices et s'étendent chacune, d'une extrémité libre (24) à un circuit électriquement conducteur (22) reliant électriquement les pointes (23) les unes aux autres.

2. Electrode selon la revendication 1, dans laquelle la plaque de matériau absorbant (4) a une épaisseur variant lorsque la plaque de matériau absorbant (4) passe de sa forme au moins partiellement expansée à sa forme au moins partiellement compressée, les pointes (23) s'étendant dans une direction de compression essentiellement parallèle cette épaisseur, et l'électrode (1) comportant un mécanisme élastique (10) permettant de déplacer les pointes (23) relativement à la plaque de matériau absorbant (4) parallèlement à la direction de compression.

3. Electrode selon la revendication 2, comportant un support (2) de forme aplatie avec deux faces principales (5, 6),
- une première face principale (5) comportant une ouverture (7) destinée à venir en vis-à-vis d'une peau animale au niveau de laquelle on souhaite recueillir la fréquence cardiaque, cette ouverture (7) permettant le passage de l'extrémité libre (24) des pointes (23), et
- une deuxième face principale (6) comportant une surface de pression (15) coopérant avec le mécanisme élastique (10) pour comprimer la plaque de matériau absorbant (4) dans la direction de compression lorsque qu'une pression est exercée sur la deuxième face principale (6) de l'électrode (1).

4. Electrode selon l'une des revendications 2 et 3, comprenant des pointes (23) périphériques, dont les extrémités libres (24) sont disposées essentiellement dans un plan perpendiculaire à la direction de compression et sur un cercle dans ce plan, aucune extrémité libre (24) de pointe (23) n'étant située hors de ce cercle.

5. Electrode selon l'une des revendications précédentes, dans laquelle chaque pointe (23) est entourée d'une portion de la plaque de matériau absorbant (4).

6. Electrode selon l'une des revendications précédentes, dans laquelle au moins l'extrémité libre (24) des pointes (23) est revêtue d'argent ou d'un alliage d'argent.

7. Electrode selon l'une des revendications 1 à 5, dans laquelle les pointes (23) et le circuit électriquement conducteur (22) auquel elles sont électriquement reliées, sont réalisés dans un polymère chargé de particules électriquement conductrices.

8. Dispositif cardio-fréquencemètre comprenant au moins une électrode (1) selon l'une des revendications précédentes et un récepteur adapté pour recevoir le signal capté par cette électrode (1).

9. Dispositif cardio-fréquencemètre selon la revendication 8, dans lequel un émetteur relié électriquement à au moins une électrode (1) et adapté pour communiquer avec le récepteur, est placé dans un boîtier (120) surélevé par rapport à une bride (110) solidaire d'une électrode (1) selon l'une des revendications 1 à 7, un espace (P) étant ainsi ménagé entre le boîtier (120) et la bride (110) pour le passage d'une sangle destinée à maintenir l'électrode (1) et le boîtier (120) sur un animal, l'électrode (1) étant placée au contact de la peau de celui-ci.

10. Procédé de mesure de la fréquence cardiaque d'un animal comprenant :
- la fourniture d'une électrode (1) selon l'une des revendications 1 à 7,
- l'imprégnation au moins partielle de la plaque de matériau absorbant (4) de cette électrode (1), avec un liquide, et
- le positionnement et le maintien de cette électrode (1) contre la peau de l'animal grâce à des moyens de maintien et de serrage.

11. Procédé de mesure de la fréquence cardiaque selon la revendication 10, comprenant une étape d'ajustement des moyens de maintien et de serrage sur l'animal, étape au cours de laquelle la plaque de matériau absorbant (4) est comprimée et libère au moins une partie du liquide qu'elle contient.

## Patentansprüche

1. Elektrode eines Herzfrequenzmessers, die enthält
- Spitzen (23),
- eine weiche Platte aus absorbierendem Werkstoff (4), die eine Kontaktfläche (26) aufweist und ihre Form elastisch verändern kann zwischen
o einer zumindest teilweise expandierten Form, in der die Platte aus absorbierendem Werkstoff (4) eine Flüssigkeit zurückhalten kann, und
o einer zumindest teilweise komprimierten Form, in der die Platte aus absorbierendem Werkstoff (4) ein geringeres Volumen einnimmt als in expandierter Form und in der zumindest ein Teil einer in der Platte aus absorbierendem Werkstoff (4), wenn er in zumindest teilweise expandierter Form ist, enthaltenen Flüssigkeit freigesetzt wird,
wobei das freie Ende (24) zumindest bestimmter der Spitzen (23) an der Kontaktfläche (26) der Platte aus absorbierendem Werkstoff (4) bündig anliegt oder über diese übersteht, wenn die Platte aus absorbierendem Werkstoff (4) in einer zumindest teilweise komprimierten Form ist und mindestens einen Teil der Flüssigkeit, die sie in ihrer zumindest teilweise expandierten Form enthält, freisetzt,
**dadurch gekennzeichnet, dass** die Spitzen (23) elektrisch leitend sind und sich je von einem freien Ende (24) zu einem elektrisch leitenden Stromkreis (22) erstrecken, der die Spitzen (23) elektrisch miteinander verbindet.

2. Elektrode nach Anspruch 1, wobei die Platte aus absorbierendem Werkstoff (4) eine Dicke hat, die variiert, wenn die Platte aus absorbierendem Werkstoff (4) von ihrer zumindest teilweise expandierten Form in ihre zumindest teilweise komprimierte Form übergeht, wobei die Spitzen (23) sich in einer Komprimierungsrichtung im Wesentlichen parallel zu dieser Dicke erstrecken, und die Elektrode (1) einen elastischen Mechanismus (10) aufweist, der es ermöglicht, die Spitzen (23) bezüglich der Platte aus absorbierendem Werkstoff (4) parallel zur Komprimierungsrichtung zu verschieben.

3. Elektrode nach Anspruch 2, die einen Träger (2) abgeflachter Form mit zwei Hauptseiten (5, 6) aufweist,
- einer ersten Hauptseite (5), die eine Öffnung (7) aufweist, die dazu bestimmt ist, sich einer Tierhaut gegenüber zu befinden, in deren Bereich die Herzfrequenz aufgefangen werden soll, wobei diese Öffnung (7) den Durchgang des freien Endes (24) der Spitzen (23) erlaubt, und
- einer zweiten Hauptseite (6), die eine Druckfläche (15) aufweist, die mit dem elastischen Mechanismus (10) zusammenwirkt, um die Platte aus absorbierendem Werkstoff (4) in der Komprimierungsrichtung zu komprimieren, wenn ein Druck auf die zweite Hauptseite (6) der Elektrode (1) ausgeübt wird.

4. Elektrode nach einem der Ansprüche 2 und 3, die Umfangsspitzen (23) enthält, deren freie Enden (24) im Wesentlichen in einer Ebene lotrecht zur Komprimierungsrichtung und auf einem Kreis in dieser Ebene angeordnet sind, wobei kein freies Ende (24) einer Spitze (23) sich außerhalb dieses Kreises befindet.

5. Elektrode nach einem der vorhergehenden Ansprüche, wobei jede Spitze (23) von einem Abschnitt der Platte aus absorbierendem Werkstoff (4) umgeben ist.

6. Elektrode nach einem der vorhergehenden Ansprüche, wobei mindestens das freie Ende (24) der Spitzen (23) mit Silber oder einer Silberlegierung bedeckt ist.

7. Elektrode nach einem der Ansprüche 1 bis 5, wobei die Spitzen (23) und der elektrisch leitende Stromkreis (22), mit dem sie elektrisch verbunden sind, aus einem mit elektrisch leitenden Partikeln angereicherten Polymer hergestellt sind.

8. Herzfrequenzmessvorrichtung, die mindestens eine Elektrode (1) nach einem der vorhergehenden Ansprüche und einen Empfänger enthält, der geeignet ist, das von dieser Elektrode (1) aufgefangene Signal zu empfangen.

9. Herzfrequenzmessvorrichtung nach Anspruch 8, wobei ein elektrisch mit mindestens einer Elektrode (1) verbundener und zur Kommunikation mit dem Empfänger geeigneter Emitter in einem Gehäuse (120) angeordnet ist, das bezüglich einer fest mit einer Elektrode (1) nach einem der Ansprüche 1 bis 7 verbundenen Lasche (110) erhöht angeordnet ist, wobei so ein Raum (P) zwischen dem Gehäuse (120) und der Lasche (110) für den Durchgang eines Gurts freigelassen wird, der dazu bestimmt ist, die Elektrode (1) und das Gehäuse (120) auf einem Tier zu halten, wobei die Elektrode (1) in Kontakt mit dessen Haut platziert wird.

10. Verfahren zum Messen der Herzfrequenz eines Tiers, das enthält:
- die Bereitstellung einer Elektrode (1) nach einem der Ansprüche 1 bis 7,
- das zumindest teilweise Tränken der Platte aus absorbierendem Werkstoff (4) dieser Elektrode (1) mit einer Flüssigkeit, und
- das Positionieren und Halten dieser Elektrode (1) gegen die Haut des Tiers mit Hilfe von Halte- und Klemmeinrichtungen.

11. Verfahren zur Messung der Herzfrequenz nach Anspruch 10, das einen Schritt der Anpassung der Halte- und Spanneinrichtungen auf dem Tier enthält, Schritt, während dessen die Platte aus absorbierendem Werkstoff (4) komprimiert wird und mindestens einen Teil der Flüssigkeit freisetzt, die sie enthält.

## Claims

1. Heart rate monitor electrode comprising
- points (23),
- a flexible sheet of absorbent material (4) comprising a contact surface (26) and that can change form elastically between
∘ an at least partially expanded form in which the sheet of absorbent material (4) can retain a liquid and
∘ an at least partially compressed form, in which the sheet of absorbent material (4) occupies a lesser volume than in its expanded form and in which at least a part of a liquid contained in the sheet of absorbent material (4), when it is in its at least partially expanded form, is released,
wherein the free end (24) of at least some of the points (23) is flush with the contact surface (26) of the sheet of absorbent material (4) or extends beyond the latter when the sheet of absorbent material (4) is in an at least partially compressed form and releases at least a part of the liquid that it contains in its at least partially expanded form,
**characterized in that** the points (23) are electrically conductive and each extend from a free end (24) to an electrically conductive circuit (22) electrically linking the points (23) to one another.

2. Electrode according to Claim 1, wherein the sheet of absorbent material (4) has a thickness that varies when the sheet of absorbent material (4) passes from its at least partially expanded form to its at least partially compressed form, the points (23) extending in a direction of compression that essentially parallels this thickness, and the electrode (1) comprising an elastic mechanism (10) making it possible to displace the points (23) relative to the sheet of absorbent material (4) parallel to the direction of compression.

3. Electrode according to Claim 2, comprising a support (2) of flattened form with two main faces (5, 6),
- a first main face (5) comprising an opening (7) intended to come opposite an animal skin where the heart rate is to be collected, this opening (7) allowing the passage of the free end (24) of the points (23), and
- a second main face (6) comprising a pressure surface (15) cooperating with the elastic mechanism (10) to compress the sheet of absorbent material (4) in the direction of compression when a pressure is exerted on the second main face (6) of the electrode (1).

4. Electrode according to one of Claims 2 and 3, comprising peripheral points (23), the free ends (24) of which are disposed essentially in a plane at right angles to the direction of compression and over a circle in this plane, no free end (24) of a point (23) being situated outside of this circle.

5. Electrode according to one of the preceding claims, wherein each point (23) is surrounded by a portion of the sheet of absorbent material (4).

6. Electrode according to one of the preceding claims, wherein at least the free end (24) of the points (23) is coated with silver or a silver alloy.

7. Electrode according to one of Claims 1 to 5, wherein the points (23) and the electrically conductive circuit (26) to which they are electrically linked, are produced in a polymer filled with electrically conductive particles.

8. Heart rate monitor device comprising at least one electrode (1) according to one of the preceding claims and a receiver suitable for receiving the signal picked up by this electrode.

9. Heart rate monitor device according to Claim 8, wherein a transmitter electrically linked to at least one electrode (1) and suitable for communicating with the receiver is placed in a housing (120) that is raised with respect to a flange (110) secured to an electrode (1) according to one of Claims 1 to 7, a space (P) being thus formed between the housing (120) and the flange (110) for the passage of a strap intended to secure the electrode (1) and the housing (120) on an animal, the electrode (1) being placed in contact with the skin thereof.

10. Method for measuring the heart rate of an animal comprising:
- the provision of an electrode (1) according to one of Claims 1 to 7,
- the at least partial impregnation of the sheet of absorbent material (4) of this electrode (1), with a liquid, and
- the positioning and securing of this electrode (1) against the skin of the animal using securing and tightening means.

11. Method for measuring heart rate according to Claim 10, comprising a step of adjustment of the securing and tightening means on the animal, a step during which the sheet of absorbent material (4) is compressed and releases at least a part of the liquid that it contains.
